# EUROPEAN PATENT SPECIFICATION

(11) **EP 3 027 739 B1**
(45) Date of publication and mention of the grant of the patent: **12.06.2019**
(21) Application number: 14842923.6
(22) Date of filing: 04.09.2014
(51) Int. Cl.: C12N 5/16, C12N 5/22, C12N 5/0735, C12N 5/074, C12N 5/0789

(54) **MODIFIED CELLS FOR PRODUCTION OF BLOOD CELLS**
MODIFIZIERTE ZELLEN ZUR ERZEUGUNG VON BLUTZELLEN
CELLULES MODIFIÉES POUR LA PRODUCTION DE CELLULES SANGUINES

(30) Priority: 06.09.2013 US 201361874539 P
(43) Date of publication of application: 08.06.2016
(73) Proprietor: Innovative Cellular Therapeutics Co., Ltd., Pudong New Area Shanghai 201203 (CN)
(72) Inventor: ZHAO, Wu, Shanghai, 201203 (CN)
(74) Representative: Abel & Imray
(86) International application number: PCT/CN2014/085952
(87) International publication number: WO 2015/032340

(56) References cited:
- WO-A1-03/020039
- WO-A1-2016/034679
- WO-A2-2009/137629
- CN-A- 1 751 130
- CN-A- 101 338 321
- CN-A- 102 850 444
- US-A- 5 811 301
- CLAUDE BAGNIS ET AL: "Elimination of blood group antigens: hope and reality", BRITISH JOURNAL OF HAEMATOLOGY, vol. 152, no. 4, 7 January 2011 (2011-01-07), pages 392-400, XP055264660, GB ISSN: 0007-1048, DOI: 10.1111/j.1365-2141.2010.08561.x
- YAZDANBAKHSH ET AL: "Molecular mechanisms underlying defective expression of blood group antigens", TRANSFUSION MEDICINE REVIEWS, GRUNE AND STRATTON, ORLANDO, FL, US, vol. 15, no. 1, 1 January 2001 (2001-01-01), pages 53-66, XP027109620, ISSN: 0887-7963 [retrieved on 2001-01-01]
- Laura Dean: "6. The Hh blood group", , 1 January 2005 (2005-01-01), XP055264636, Retrieved from the Internet: URL:http://www.ncbi.nlm.nih.gov/books/NBK2 268/pdf/Bookshelf_NBK2268.pdf [retrieved on 2016-04-12]
- YOUNG-HOON KIM ET AL: "Rh D blood group conversion using transcription activator-like effector nucleases", NATURE COMMUNICATIONS, vol. 6, 16 June 2015 (2015-06-16), page 7451, XP055264675, DOI: 10.1038/ncomms8451
- CLAUDE BAGNIS: "Silencing and overexpression of human blood group antigens in transfusion: Paving the way for the next steps", BLOOD REVIEWS, vol. 29, no. 3, 1 May 2015 (2015-05-01), pages 163-169, XP055264664, AMSTERDAM, NL ISSN: 0268-960X, DOI: 10.1016/j.blre.2014.10.004
- SANTOSH KUMAR PATNAIK: 'BGMUT: NCBI dbRBC database of allelic variations of genes encoding antigens of blood group systems' NUCLEIC ACIDS RESEARCH vol. 40, 13 November 2011, pages 1023 - 1029, XP055261338

## Description

### BACKGROUND

Blood transfusion therapies generally involve transplanting red blood cells (RBCs), platelets, and etc. Although blood transfusions may use one's own blood (autologous transfusion), it is more common to use someone else's (allogeneic or homologous transfusion). Using another's blood starts with donation of blood, and blood is most commonly donated as whole blood intravenously and collecting it with an anticoagulant. However, blood transfusions using others' blood may be associated with unpredictable adverse results, many of which may be grouped as immunological or infectious reactions. For example, immunological reactions may include acute hemolytic reactions, delayed hemolytic reactions, febrile nonhemolytic reactions, etc. In addition, donated whole blood may include hazardous viruses or prions, which are difficult to prevent completely. Therefore, there is a need in the art for approaches and techniques that minimize or prevent unpredictable adverse results associated with blood transfusions.

Bagnis, C., et al, British Journal of Haematology, 2011, Vol 152(4), pages 392 - 400 provides a review of techniques for elimination of blood group antigens. CN 102 850 444 discloses a pair of transcription activator like effector nucleases (TALEN) obtained by respective fusion of a pair of DNA recognins with two heterogenous subunits of a Fok1 DNA endonuclease, that can specifically recognize two adjacent loci on human RHD or RHCE gene exon1. US 5 811 301 discloses a method for *in vitro* production of clinically useful quantities of single species of mature, differentiated human blood cells in which human pluripotent hematopoietic stem cells are incubated in a bioreactor in a growth medium also containing specific combinations of recombinant human growth and maturation promoting polypeptide factors that expand stem cell cultures and promote the maturation and differentiation of stem cells into single species of erythroid, thrombocytic or granulocytic human blood cells, and harvesting the mature cells. WO 2009/137629 discloses methods for generating enucleated erythroid cells using pluripotent stem cells. Yazdanbakhsh, et al, Transfusion Medicine Reviews, 2001, Vol. 15(1), pages 53 - 66 provides a review of molecular mechanisms underlying defective expression of blood group antigens. Laura Dean "6. The Hh blood group", 2005, retrieved from the Internet: URL:http://www.ncbi.nlm.nih.gov/books/NBK2268/pdf/Bookshelf_NBK2268.p

### SUMMARY

Embodiments of the present disclosure relate to a method for generating a modified stem cell. The method includes culturing in a culture media modified stem cells containing a complete or partial gene deletion of gene H of the blood group antigen (BGA) biosynthesis or transportation pathway that renders the product of gene H non-functional or having reduced function with respect to the synthesis of the ABO blood group antigen, wherein the method comprises:
providing collected stem cells containing a target polynucleotide associated with gene H of the blood group antigen blood group antigen (BGA) biosynthesis or transportation pathway;
introducing a transcription activator-like (TAL) effector nuclease into the stem cells to generate the modified stem cells;
isolating the modified stem cells; and culturing in a culture media the modified stem cells containing a complete or partial gene deletion of gene H;
and wherein multiple TAL effector repeat sequences bind to the target polynucleotide of gene H of the BGA biosynthesis or transportation pathway.

Embodiments of the present disclosure also relate to a method for generating blood substitutes. The method includes generating modified stem cells according to the method of the present disclosure, culturing in a culture media the modified stem cells containing a complete or partial gene deletion of gene H of the blood group antigen (BGA) biosynthesis or transportation pathway; and
differentiating the modified stem cells to generate a modified blood cell that has a reduced amount of the ABO blood group antigen as compared to a corresponding wild-type cell.

In some embodiments of the methods of the present disclosure, the modified cell may include a genetically modified hematopoietic stem cell or a genetically modified erythroid progenitor cell. In certain embodiments, the modified cell may include a genetically modified stem cell. In particular embodiments, the modified cell may include a genetically modified embryonic stem cell or a genetically modified induced pluripotent stem cell.

In some embodiments of the methods of the present disclosure, the modified cell may be differentiated into a blood cell. In certain embodiments, the blood cell may have a reduced amount of the ABO blood group antigen as compared to a corresponding wild-type blood cell. In particular embodiments, the blood cell may have reduced expression of gene H of the BGA biosynthesis or transportation pathway as compared to a corresponding wild-type blood cell. For example, the blood cell accumulates a reduced amount of the ABO BGA in the modified cell as compared to a corresponding wild-type cell. For example, the blood cell has a reduced amount of the ABO BGA on the modified cell as compared to a corresponding wild-type cell. For example, the blood cell has a reduced amount of the ABO BGA associated with a membrane of the modified cell as compared to a corresponding wild-type cell.

In some embodiments of the methods of the present disclosure, the blood cell derived from the modified cell has decreased immunogenicity as compared to a corresponding wild-type cell in response to a blood transfusion. In certain embodiments, the decreased immunogenicity comprises a deceased level of immunological responses induced by the modified cell as compared to the corresponding wild-type cell. For example, the level of immunological responses induced by the modified stem cell or cells is substantially less than a level of immunological responses induced by the corresponding wild-type cell. In certain embodiments, the level of immunological responses induced by the modified stem cell or cells is measured at certain time post-initiation of an injection of the modified cell.

In some embodiments of the methods of the present disclosure, the BGA comprises an ABO blood group antigen.

This Summary is provided to introduce a selection of concepts in a simplified form that are further described below in the Detailed Description.

### BRIEF DESCRIPTION OF THE DRAWINGS

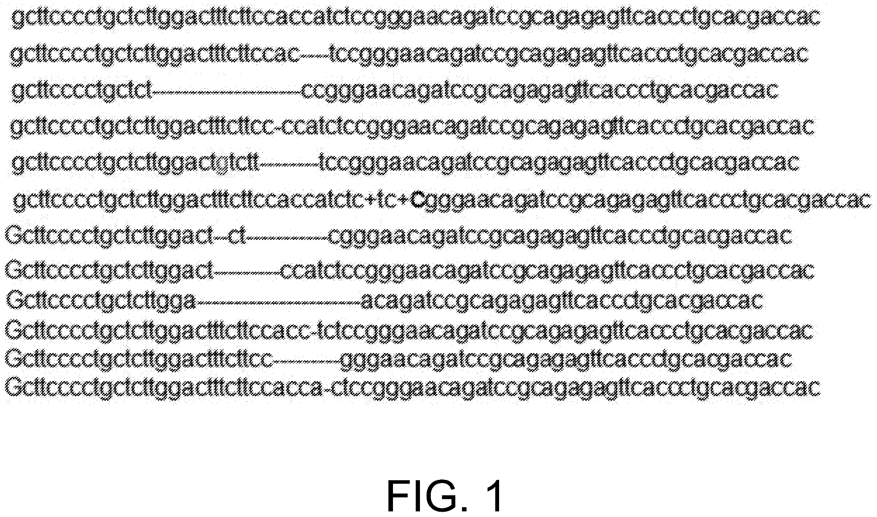

FIG. 1 illustrates modified cells using TALENs. The first line is standard sequences and the other lines are mutated sequences. The broken lines cover the deletion caused by mutation.
FIG. 2 shows chromatogram of sequencing results. The peaks on the right side of black straight line overlapped with each others, indicating heterogeneity of genomic sequences which is caused by frame-shift mutation.

### DETAILED DESCRIPTION

### Overview

Embodiments of the present disclose contemplate a user of genetically modified stem cells as resources for producing blood substitutes, such as red blood cells (RBCs), plasma, serum, etc. The present disclosure relates, in part, to the demonstration that stem cells, such as embryonic stem cells, induced pluripotent stem cells, etc, can be genetically modified and differentiated into blood substitutes such as RBCs, white blood cells, and etc. For example, as shown in the accompanying Example, a genetically modified cell may be modified to contain a complete or partial gene deletion of one or more genes of a blood group antigen (BGA) biosynthesis or transportation pathway. Examples of the BGA may include an Rh blood group antigen or an ABO blood group antigen.

### Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

The term "biologically active fragment", as applied to fragments of a reference polynucleotide or polypeptide sequence, refers to a fragment that has at least 0.1, 0.5, 1, 2, 5, 10, 12, 14, 16, 18, 20, 22, 24, 26, 28, 30, 35, 40, 45, 50, 55, 60, 65, 70, 75, 80, 85, 90, 95, 96, 97, 98, 99, 100, 110, 120, 150, 200, 300, 400, 500, 600, 700, 800, 900, 1000% or more of the activity of a reference sequence. The term "reference sequence" refers generally to a nucleic acid coding sequence, or amino acid sequence, to which another sequence is being compared. All sequences provided in the Sequence Listing are also included as reference sequences.

The term "biologically active variant", as applied to variants of a reference polynucleotide or polypeptide sequence, refers to a variant that has at least 0.1, 0.5, 1, 2, 5, 10, 12, 14, 16, 18, 20, 22, 24, 26, 28, 30, 35, 40, 45, 50, 55, 60, 65, 70, 75, 80, 85, 90, 95, 96, 97, 98, 99, 100, 110, 120, 150, 200, 300, 400, 500, 600, 700, 800, 900, 1000% or more of the activity (e.g., an enzymatic activity) of a reference sequence. The term "reference sequence" refers generally to a nucleic acid coding sequence, or amino acid sequence, to which another sequence is being compared. The term "variant" encompasses biologically active variants, which may also be referred to as functional variants.

Included within the scope of the present invention are biologically active fragments of at least 18, 19, 20, 21, 22, 23, 24, 25, 26, 27, 28, 29, 30, 40, 50, 60, 70, 80, 90, 100, 120, 140, 160, 180, 200, 220, 240, 260, 280, 300, 320, 340, 360, 380, 400, 500, 600 or more contiguous nucleotides or amino acid residues in length, including all integers in between, which comprise or encode a polypeptide having an activity of a reference polynucleotide or polypeptide. Representative biologically active fragments and variants generally participate in an interaction, e.g., an intra-molecular or an inter-molecular interaction. An inter-molecular interaction can be a specific binding interaction or an enzymatic interaction. Examples of enzymatic interactions or activities include, without limitation, a glycosyltransferase activity a fucosyltransferase activity, and other enzymatic activities described herein.

The term "blood cell" refers to at least one of red blood cells (RBCs), white blood cells, platelets, or other blood cells. For example, RBCs are blood cells of delivering oxygen (O₂) to the body tissues via the blood flow through the circulatory system. White blood cells are cells of the immune system involved in defending the body against both infectious disease and foreign materials. Platelets are cells that are involved in hemostasis, leading to the formation of blood clots.

The term "blood substitute" refers to a composition useable to perform, mimic and/or fulfill one or more functions of at least one of red blood cells (RBCs), plasma, serum, or other constituents of biological blood. In some embodiments, the blood substitute may be derived from stem cells. For example, embryonic stem cells may be differentiated into the hematopoietic lineage stem cell, which may be further differentiated into erythroid progenitor cells, myeloid, B cell lineages, and etc. to produce, for example, RBCs *in vitro.* These *in vitro* produced RBCs may be used for blood transfusion as blood substitutes.

The term "blood group antigen" refers to a substance (e.g., sugars, proteins, etc.) attached to various components in the red blood cell membrane. In some embodiments, the blood group antigen may include a protein or peptide antigens present on the surface of a red blood cell. For example, an Rh blood group antigen may include a D antigen, a C antigen, a c antigen, an E antigen, an e antigen, or any other related Rh antigens. In some embodiments, blood group antigens may include a sugar antigen (e.g., glycan or carbohydrate) that is produced by a series of reactions in which enzymes catalyze the transfer of corresponding sugar units. For example, an ABO blood group antigen may include A antigen, B antigen, A1 antigen, or any other related ABO antigens.

The "anti-(blood group antigen) antibody" refers an antibody which specifically recognizes and binds to an antigen present on the surface of a red blood cell. For example, an anti-Rh antibody may include an anti-D antibody, an anti-C antibody, an anti-c antibody, an anti-E antibody, an anti-e antibody, or any other related Rh antibody. For example an anti-ABO antibody may include anti-A, anti-B, anti-A1, or any other related ABO antibody.

In addition to the two major blood group systems (i.e., ABO and Rh) the following blood group antigen systems are known, and are also considered to be within the scope of the present disclosure: MNSsU, P1, Lutheran, Kell, Lewis, Duffy, Kidd, Diego, Cartwright, Dombrock, Colton, Scianna, Xga, I/i, Augustine, Cromer, Ena, Gerbich, Gregory, Holley, jacobs, Joseph, Lngereis, Oka, Vel, Chido, Rodgers, Cost-Stirling, York, Knops-Helgeson, McCoy, John Milton Hagen, Ahonen, Batty, Biles, Bishop, Box, Chra, Dantu, Froese, Good, Griffiths, Heibel, Hey, Hov, Hunt, Jensen, Jna, Lewis II, Livesey, Mitchell, Moen, Orriss, Peters, Radin, Redelberger, Reid, Rosennlund, Swann, Torkiidsen, Traversu, Webb, Wright, Wulfsberg, and Bg. Each of these systems may have several antigens and phenotypes, but all have at least one known antigen (e.g., Lewis has two antigens and 3 phenotypes).

By "coding sequence" is meant any nucleic acid sequence that contributes to the code for the polypeptide product of a gene. By contrast, the term "non-coding sequence" refers to any nucleic acid sequence that does not contribute to the code for the polypeptide product of a gene.

Throughout this specification, unless the context requires otherwise, the words "comprise", "comprises" and "comprising" will be understood to imply the inclusion of a stated step or element or group of steps or elements but not the exclusion of any other step or element or group of steps or elements.

By "consisting of" is meant including, and limited to, whatever follows the phrase "consisting of." Thus, the phrase "consisting of" indicates that the listed elements are required or mandatory, and that no other elements may be present.

By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The terms "complementary" and "complementarity" refer to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

By "corresponds to" or "corresponding to" is meant (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

By "derivative" is meant a polypeptide that has been derived from the basic sequence by modification, for example by conjugation or complexing with other chemical moieties (e.g., pegylation) or by post-translational modification techniques as would be understood in the art. The term "derivative" is also meant a chemical compounds (e.g., sugars) that has been derived from the basic structure by modification, for example, by conjugation or complexing with other chemical moieties (*e.g*., glycosylation). The term "derivative" also includes within its scope alterations that have been made to a parent sequence/structure including additions or deletions that provide for functionally equivalent molecules.

By "enzyme reactive conditions" it is meant that any necessary conditions are available in an environment (i.e., such factors as temperature, pH, lack of inhibiting substances) which will permit the enzyme to function. Enzyme reactive conditions can be either in vitro, such as in a test tube, or in vivo, such as within a cell.

As used herein, an "EcoR I " (or "BamH I") includes an enzyme that cuts DNA at specific recognition nucleotide sequences.

As used herein, the terms "function" and "functional" and the like refer to a biological, enzymatic, or therapeutic function.

By "gene" is meant a unit of inheritance that occupies a specific locus on a chromosome and consists of transcriptional and/or translational regulatory sequences and/or a coding region and/or non-translated sequences (i.e., introns, 5' and 3' untranslated sequences).

"Homology" refers to the percentage number of amino acids that are identical or constitute conservative substitutions. Homology may be determined using sequence comparison programs such as GAP (Deveraux et al., 1984, Nucleic Acids Research 12, 387-395) which is incorporated herein by reference. In this way sequences of a similar or substantially different length to those cited herein could be compared by insertion of gaps into the alignment, such gaps being determined, for example, by the comparison algorithm used by GAP.

The term "host cell" includes an individual cell or cell culture which can be or has been a recipient of any recombinant vector(s) or isolated polynucleotide of the invention. Host cells include progeny of a single host cell, and the progeny may not necessarily be completely identical (in morphology or in total DNA complement) to the original parent cell due to natural, accidental, or deliberate mutation and/or change. A host cell includes cells transfected or infected in vivo or in vitro with a recombinant vector or a polynucleotide of the invention. A host cell which comprises a recombinant vector of the invention is a recombinant host cell.

The term "stem cell" refers to any of certain types of cell which have the capacity for self-renewal and the ability to differentiate into other kind(s) of cell. For example, a stem cell gives rise either to two daughter stem cells (as occurs in vitro with embryonic stem cells in culture) or to one stem cell and a cell that undergoes differentiation (as occurs e.g. in hematopoietic stem cells, which give rise to blood cells). Different categories of stem cell may be distinguished on the basis of their origin and/or on the extent of their capacity for differentiation into other types of cell. For example, stem cell may include embryonic stem (ES) cells (i.e., pluripotent stem cells), somatic stem cells, Induced pluripotent stem cells, and any other types stem cells.

Pluripotent embryonic stem cells can be found in the inner cell mass of a BLASTOCYST, and have high innate capacity for differentiation. For example, pluripotent embryonic stem cells may have the potential to form any type of cell in the body. When grown in vitro for long periods of time, ES cells maintain pluripotency: progeny cells retain the potential for multilineage differentiation.

Somatic stem cells may include the fetal stem cells (from the fetus) and adult stem cells (found in various tissues, such as bone marrow). These cells have been regarded as having a capacity for differentiation lower than that of the pluripotent ES cells - with the capacity of fetal stem cells being greater than that of adult stem cells; they apparently differentiate into only a limited range of types of cell and have been described as multipotent. The 'tissue-specific' stem cells normally give rise to only one type of cell. For example, embryonic stem cells may be differentiated into blood stem cells (e.g., Hematopoietic stem cells (HSCs)), which may be further differentiated into various blood cells (e.g., red blood cells, platelets, white blood cells, etc.).

Induced pluripotent stem cells (i.e., iPS cells or iPSCs) may include a type of pluripotent stem cell artificially derived from a non-pluripotent cell (e.g., an adult somatic cell) by inducing a expression of specific genes. Induced pluripotent stem cells are similar to natural pluripotent stem cells, such as embryonic stem (ES) cells, in many aspects, such as the expression of certain stem cell genes and proteins, chromatin methylation patterns, doubling time, embryoid body formation, teratoma formation, viable chimera formation, and potency and differentiability. Induced pluripotent cells may be made from adult stomach, liver, skin cells and blood cells.

By "isolated" is meant material that is substantially or essentially free from components that normally accompany it in its native state. For example, an "isolated polynucleotide", as used herein, refers to a polynucleotide, which has been purified from the sequences which flank it in a naturally-occurring state, e.g., a DNA fragment which has been removed from the sequences that are normally adjacent to the fragment. Alternatively, an "isolated peptide" or an "isolated polypeptide" and the like, as used herein, refer to in vitro isolation and/or purification of a peptide or polypeptide molecule from its natural cellular environment, and from association with other components of the cell.

The terms "modulating" and "altering" include "increasing" and "enhancing" as well as "decreasing" or "reducing," typically in a statistically significant or a physiologically significant amount or degree relative to a control. In specific embodiments, immunological rejection associated with transplantation of the blood substitutes is decreased relative to an unmodified or differently modified stem cell by at least 10%, at least 15%, at least 20%, at least 25%, at least 30%, at least 40%, at least 50%, at least 60%, at least 70%, at least 80%, at least 90%, at least 100%, at least 150%, at least 200%, at least 300%, at least 400%, at least 500%, or at least 1000%.

An "increased" or "enhanced" amount is typically a "statistically significant" amount, and may include an increase that is 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein.

Immune rejection can be measured according to techniques known in the art, such as grafts transplantation, Elispot, etc.

A "decreased" or "reduced" or "lesser" amount is typically a "statistically significant" amount, and may include a decrease that is 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein. For example, "In specific embodiments, immunological rejection associated with transplantation of the blood substitutes is decreased relative to an unmodified or differently modified stem cell by at least 10%, at least 15%, at least 20%, at least 25%, at least 30%, at least 40%, at least 50%, at least 60%, at least 70%, at least 80%, at least 90%, at least 100%, at least 150%, at least 200%, at least 300%, at least 400%, at least 500%, or at least 1000%.

By "obtained from" is meant that a sample such as, for example, a polynucleotide or polypeptide is isolated from, or derived from, a particular source, such as a desired organism or a specific tissue within a desired organism. "Obtained from" can also refer to the situation in which a polynucleotide or polypeptide sequence is isolated from, or derived from, a particular organism or tissue within an organism. For example, a polynucleotide sequence encoding a reference polypeptide described herein may be isolated from a variety of prokaryotic or eukaryotic organisms, or from particular tissues or cells within certain eukaryotic organism.

The term "operably linked" as used herein means placing a gene under the regulatory control of a promoter, which then controls the transcription and optionally the translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e. the gene from which the genetic sequence or promoter is derived. As is known in the art, some variation in this distance can be accommodated without loss of function. Similarly, the preferred positioning of a regulatory sequence element with respect to a heterologous gene to be placed under its control is defined by the positioning of the element in its natural setting; i.e., the gene from which it is derived. "Constitutive promoters" are typically active, i.e., promote transcription, under most conditions. "Inducible promoters" are typically active only under certain conditions, such as in the presence of a given molecule factor (e.g., IPTG) or a given environmental condition (e.g., particular CO2 concentration, nutrient levels, light, heat). In the absence of that condition, inducible promoters typically do not allow significant or measurable levels of transcriptional activity. For example, inducible promoters may be induced according to temperature, pH, a hormone, a metabolite (e.g., lactose, mannitol, an amino acid), light (e.g., wavelength specific), osmotic potential (e.g., salt induced), a heavy metal, or an antibiotic. Numerous standard inducible promoters will be known to one of skill in the art.

The recitation "polynucleotide" or "nucleic acid" as used herein designates mRNA, RNA, cRNA, rRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA and RNA.

The terms "polynucleotide variant" and "variant" and the like refer to polynucleotides displaying substantial sequence identity with a reference polynucleotide sequence or polynucleotides that hybridize with a reference sequence under stringent conditions that are defined hereinafter. These terms also encompass polynucleotides that are distinguished from a reference polynucleotide by the addition, deletion or substitution of at least one nucleotide. Accordingly, the terms "polynucleotide variant" and "variant" include polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide, or has increased activity in relation to the reference polynucleotide (i.e., optimized). Polynucleotide variants include, for example, polynucleotides having at least 50% (and at least 51% to at least 99% and all integer percentages in between, e.g., 90%, 95%, or 98%) sequence identity with a reference polynucleotide sequence described herein. The terms "polynucleotide variant" and "variant" also include naturally-occurring allelic variants and orthologs that encode these enzymes.

With regard to polynucleotides, the term "exogenous" refers to a polynucleotide sequence that does not naturally-occur in a wild-type cell or organism, but is typically introduced into the cell by molecular biological techniques. Examples of exogenous polynucleotides include vectors, plasmids, and/or man-made nucleic acid constructs encoding a desired protein. With regard to polynucleotides, the term "endogenous" or "native" refers to naturally-occurring polynucleotide sequences that may be found in a given wild-type cell or organism. Also, a particular polynucleotide sequence that is isolated from a first organism and transferred to second organism by molecular biological techniques is typically considered an "exogenous" polynucleotide with respect to the second organism. In specific embodiments, polynucleotide sequences can be "introduced" by molecular biological techniques into a microorganism that already contains such a polynucleotide sequence, for instance, to create one or more additional copies of an otherwise naturally-occurring polynucleotide sequence, and thereby facilitate overexpression of the encoded polypeptide.

The recitations "mutation" or "deletion," in relation to the genes of a "blood group antigen biosynthesis or storage pathway" refer generally to those changes or alterations in a stem cell that render the product of that gene non-functional or having reduced function with respect to the synthesis and/or storage of the blood group antigen. Examples of such changes or alterations include nucleotide substitutions, deletions, or additions to the coding or regulatory sequences of a targeted gene (e.g., *ABO*, Gene *H*, *RHD*, *XK*, *FUT*, etc), in whole or in part, which disrupt, eliminate, down-regulate, or significantly reduce the expression of the polypeptide encoded by that gene, whether at the level of transcription or translation, and/or which produce a relatively inactive (e.g., mutated or truncated) or unstable polypeptide. Techniques for producing such alterations or changes, such as by recombination with a vector having a selectable marker, are exemplified herein and known in the molecular biological art. In particular embodiments, one or more alleles of a gene, e.g., two or all alleles, may be mutated or deleted within a stem cell.

The "deletion" of a targeted gene may also be accomplished by targeting the mRNA of that gene, such as by using various antisense technologies (e.g., antisense oligonucleotides and siRNA) known in the art. Accordingly, targeted genes may be considered "non-functional" when the polypeptide or enzyme encoded by that gene is not expressed by the modified cell, or is expressed in negligible amounts, such that the modified cell produces or accumulates less of the polypeptide or enzyme product than an unmodified or differently modified stem cell.

In certain aspects, a targeted gene may be rendered "non-functional" by changes or mutations at the nucleotide level that alter the amino acid sequence of the encoded polypeptide, such that a modified polypeptide is expressed, but which has reduced function or activity with respect to its enzymatic activity (e.g., a glycosyltransferase activity, a fucosyltransferase activity, etc.), whether by modifying that polypeptide's active site, its cellular localization, its stability, or other functional features apparent to a person skilled in the art. Such modifications to the coding sequence of a polypeptide involved in the blood group antigen biosynthesis and/or storage pathway may be accomplished according to known techniques in the art, such as site directed mutagenesis at the genomic level and/or natural selection (i.e., directed evolution) of a given stem cell.

"Polypeptide," "polypeptide fragment," "peptide" and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers. In certain aspects, polypeptides may include enzymatic polypeptides, or "enzymes", which typically catalyze (i.e., increase the rate of) various chemical reactions.

The recitation polypeptide "variant" refers to polypeptides that are distinguished from a reference polypeptide sequence by the addition, deletion or substitution of at least one amino acid residue. In certain embodiments, a polypeptide variant is distinguished from a reference polypeptide by one or more substitutions, which may be conservative or non-conservative. In certain embodiments, the polypeptide variant comprises conservative substitutions and, in this regard, it is well understood in the art that some amino acids may be changed to others with broadly similar properties without changing the nature of the activity of the polypeptide. Polypeptide variants also encompass polypeptides in which one or more amino acids have been added or deleted, or replaced with different amino acid residues.

The term "reference sequence" refers generally to a nucleic acid coding sequence, or amino acid sequence, to which another sequence is being compared. All polypeptide and polynucleotide sequences described herein are included as references sequences.

The present invention contemplates the use in the methods described herein of variants of full-length enzymes or reference sequences, for instance, those having a glycosyltransferase activity a fucosyltransferase activity, etc., among other reference sequences described herein, truncated fragments of these full-length enzymes and polypeptides, variants of truncated fragments, as well as their related biologically active fragments. Typically, biologically active fragments of a polypeptide may participate in an interaction, for example, an intra-molecular or an inter-molecular interaction. An inter-molecular interaction can be a specific binding interaction or an enzymatic interaction (e.g., the interaction can be transient and a covalent bond is formed or broken).

Biologically active fragments of a polypeptide/enzyme having a selected activity include peptides comprising amino acid sequences sufficiently similar to, or derived from, the amino acid sequences of a (putative) full-length reference polypeptide sequence. Typically, biologically active fragments comprise a domain or motif with at least one activity of a reference sequence or enzyme described herein, or a polypeptide associated with the blood group antigen biosynthesis and/or storage pathway, and may include one or more (and in some cases all) of the various active domains. A biologically active fragment of such polypeptides can be a polypeptide fragment which is, for example, 10, 11, 12, 13, 14, 15, 16, 17, 18, 19, 20, 21, 22, 23, 24, 25, 26, 27, 28, 29,30, 40, 50, 60, 70, 80, 90, 100, 110, 120, 130, 140, 150, 160, 170, 180, 190, 200, 220, 240, 260, 280, 300, 320, 340, 360, 380, 400, 450, 500, 600 or more contiguous amino acids, including all integers in between, of a reference polypeptide sequence. In certain embodiments, a biologically active fragment comprises a conserved enzymatic sequence, domain, or motif, as described elsewhere herein and known in the art. Suitably, the biologically-active fragment has no less than 1%, 10%, 25%, 50% of an activity of the wild-type polypeptide from which it is derived.

The recitations "sequence identity" or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions,
dividing the number of matched positions by the total number of positions in the window of comparison (i.e., the window size), and multiplying the result by 100 to yield the percentage of sequence identity. Included are nucleotides and polypeptides having at least 50%, 55%, 60%, 65%, 70%, 75%, 80%, 85%, 90%, 95%, 97%, 98%, 99% or 100% sequence identity to any of the reference sequences described herein (see, e.g., Sequence Listing), typically where the polypeptide variant maintains at least one biological activity of the reference polypeptide. Terms used to describe sequence relationships between two or more polynucleotides or polypeptides include "reference sequence", "comparison window", "sequence identity", "percentage of sequence identity" and "substantial identity". A "reference sequence" is at least 12 but frequently 15 to 18 and often at least 25 monomer units, inclusive of nucleotides and amino acid residues, in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of at least 6 contiguous positions, usually 50 to 100, more usually 100 to 150 in which a sequence is compared to a reference sequence of the same number of contiguous positions after the two sequences are optimally aligned. The comparison window may comprise additions or deletions (i.e., gaps) of 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e., resulting in the highest percentage homology over the comparison window) generated by any of the various methods selected. Reference also may be made to the BLAST family of programs as for example disclosed by Altschul et al., 1997, Nucl. Acids Res. 25:3389. A detailed discussion of sequence analysis can be found in Unit 19.3 of Ausubel et al., "Current Protocols in Molecular Biology", John Wiley & Sons Inc, 1994-1998, Chapter 15.

By "statistically significant," it is meant that the result was unlikely to have occurred by chance. Statistical significance can be determined by any method known in the art. Commonly used measures of significance include the p-value, which is the frequency or probability with which the observed event would occur, if the null hypothesis were true. If the obtained p-value is smaller than the significance level, then the null hypothesis is rejected. In simple cases, the significance level is defined at a p-value of 0.05 or less.

"Substantially" or "essentially" means nearly totally or completely, for instance, 95%, 96%, 97%, 98%, 99% or greater of some given quantity.

"Transformation" refers to the permanent, heritable alteration in a cell resulting from the uptake and incorporation of foreign DNA into the host-cell genome; also, the transfer of an exogenous gene from one organism into the genome of another organism.

By "vector" is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, i.e., a vector that exists as an extra-chromosomal entity, the replication of which is independent of chromosomal replication, e.g., a linear or closed circular plasmid, an extra-chromosomal element, a mini-chromosome, or an artificial chromosome. The vector can contain any means for assuring self-replication. Alternatively, the vector can be one which, when introduced into the host cell, is integrated into the genome and replicated together with the chromosome(s) into which it has been integrated. Such a vector may comprise specific sequences that allow recombination into a particular, desired site of the host chromosome. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. In the present case, the vector is preferably one which is operably functional in a stem cell. The vector can include a reporter gene, such as a green fluorescent protein (GFP), which can be either fused in frame to one or more of the encoded polypeptides, or expressed separately. The vector can also include a selection marker such as an antibiotic resistance gene that can be used for selection of suitable transformants.

The term "wild-type" refers to a gene or gene product that has the characteristics of that gene or gene product when isolated from a naturally-occurring source. A wild-type gene or gene product (e.g., a polypeptide) is that which is most frequently observed in a population and is thus arbitrarily designed in the "normal" or "wild-type" form of the gene.

### Illustrated Embodiments

Described herein is a genetically modified cell containing a complete or partial gene deletion of gene H of the blood group antigen (BGA) biosynthesis or transportation pathway.

Embodiments of the present disclosure relate to a method for generating a modified stem cell. The method includes culturing in a culture media modified stem cells containing a complete or partial gene deletion of gene H of the blood group antigen (BGA) biosynthesis or transportation pathway.

Embodiments of the present disclosure also relate to a method for generating blood substitutes. The method includes culturing in a culture media modified stem cells containing a complete or partial gene deletion of gene H of the blood group antigen (BGA) biosynthesis or transportation pathway. The modified stem cells may be then differentiated to generate a modified blood cell that has a reduced amount of the ABO BGA as compared to a corresponding wild-type cell.

In some embodiments, the modified cell may include a genetically modified hematopoietic stem cell or a genetically modified erythroid progenitor cell. In certain embodiments, the modified cell may include a genetically modified stem cell. In particular embodiments, the modified cell may include a genetically modified embryonic stem cell or a genetically modified induced pluripotent stem cell.

The complete or partial gene deletion of gene H is implemented by transcription activator-like effector nucleases (TALENs) related techniques. TALEN is a type of fused protein, and may comprise TAL of genus Xanthomonas and FokI. TAL (transcription activator-like) is composed with several modules arranged in certain order to recognize corresponding sequences, ensuring the accurate cleavage (See Tables. 1 and 2). TALENs may be used in genetic editing in different species as zebra fish, swine, cow, mouse, rat and cells of human being. Human embryonic stem cells (hESCs) may be genetically edited by TALEN technique. For example, genes crucial for classification of blood group may be nullified by TALEN technique in hESCs, which are then differentiated into erythrocytes without expression of essential antigen for classification of blood group. This may allow the adaptability of corresponding blood product to blood recipients of many blood groups. The proliferative and expansive characteristics of human embryonic may solve resource issues. Meanwhile the production method of embryonic stem cell differentiation may avoid or mitigate risk associated with contamination of pathogens.

In some embodiments, the modified cell may be differentiated into a blood cell. In certain embodiments, the blood cell may have a reduced amount of the ABO BGA as compared to a corresponding wild-type blood cell. In particular embodiments, the blood cell may have reduced expression of gene H of the BGA biosynthesis or transportation pathway as compared to a corresponding wild-type blood cell. For example, the blood cell accumulates a reduced amount of the ABO BGA in the modified cell as compared to a corresponding wild-type cell. For example, the blood cell has a reduced amount of the ABO BGA

on the modified cell as compared to a corresponding wild-type cell. For example, the blood cell has a reduced amount of the ABO BGA associated with a membrane of the modified cell as compared to a corresponding wild-type cell.

**Table 1**

| | |
|---|---|
| | EQVVAIASNIGGKQALETVQRLLPVLCQAHG |
| | EQVVAIASNGGGKQALETVQRLLPVLCQAHG |
| | EQVVAIASHDGGKQALETVQRLLPVLCQAHG |
| | EQVVAIASNNGGKQALETVQRLLPVLCQAHG |

**Table 2**

| | |
|---|---|
| A | CACTCCGGAACAAGTGGTCGCAATCGCCTCCAACATTGGCGGGAAACAGGCACTCGAGACTG CAGCGCCTGCTTCCCGTGCTGTGCCAAGCGCACGGT |
| T | GACCCCAGAGCAGGTCGTGGCCATTGCCTCGAATGGAGGGGGCAAACAGGCGTTGGAAACC ACAACGATTGCTGCCGGTGCTTTGTCAGGCACACGGC |
| C | GACCCCAGAGCAGGTCGTGGCGATCGCAAGCCACGACGGAGGAAAGCAAGCCTTGGAAACA ACAGAGGCTGTTGCCTGTGCTTTGTCAGGCACACGGC |
| G | CACTCCGGAACAAGTGGTCGCAATCGCGAGCAATAACGGCGGAAAACAGGCTTTGGAAACG GCAGAGGCTCCTTCCAGTGCTGTGCCAAGCGCACGGT |

In some embodiments, the blood cell derived from the modified cell has decreased immunogenicity as compared to a corresponding wild-type cell in response to a blood transfusion. In certain embodiments, the decreased immunogenicity comprises a deceased level of immunological responses induced by the modified cell as compared to the corresponding wild-type cell. For example, the level of immunological responses induced by the modified stem cell or cells is less than a level of immunological responses induced by the corresponding wild-type cell. In certain embodiments, the level of immunological responses induced by the modified stem cell or cells is measured at certain time post-initiation of an injection of the modified cell.

In some embodiments, the BGA comprises an ABO blood group antigen. Different types of antigens located in/on or secreted by red blood may
determine several systems of human blood groups (See Table. 3). Blood transfusion between people of different blood group may cause severe outcomes such as hemolysis. ABO and Rh blood group systems are two generally accepted blood type systems. ABO blood system may include type A, B, AB, O and rare Bombay type. These types are different based on antigens located in red blood cells and platelets. For example, determination of A antigen, B antigen and combination of two in a red blood cell indicate blood type A, B and AB respectively. H antigen is precursor for synthesis of A and B antigens, and preservation of H antigen give rise to blood group O. For rare type of Bombay, H antigen was not synthesized.

Rh system contains types of Rh- and Rh+, distinguished by existence of Rh D antigen in blood cells. The principle of compatibility should be strictly followed during blood transfusions. Otherwise, different types of antigens or antibodies in blood cells from donors would cause immunological reactions between antibodies and antigen, culminating in hemolysis and even death of recipients. The frequency of Rh- is 1% or lower in US and Asia, while the ratio of Bombay type is less than 0.0004%.

The various embodiments described above can be combined to provide further embodiments. Aspects of the embodiments can be modified, if necessary to employ concepts of the various patents, applications and publications to provide yet further embodiments.

**Table 3**

| Blood Group System | Antigen | Subtypes | Crux Gene | Rare Type |
|---|---|---|---|---|
| ABO | A,B,O/H | A,B,AB,O, | Gene H | Bombay type |
| Rh | RhD | Rh-, Rh+ | RHD | RhD- |
| Kell | K1,K2 | K1,K2,K0 | XK | KO |
| MNS | M,N,S,s,U | M+,N+,MN+,S+,s+,U+ | GPA,GPB(coding S | S-s- |
| Kidd | Jka,Jkb | Jk1,Jk2,Jk3 | SLC14A1 | Jk3 |
| Duffy | Fya,Fyb,Fy3 | Fy(a+b+),Fy(a+b-),Fy(a-b+),Fy(a-b-) | gp-Fy | Fy(a-b+) (In Chinese<1%) |
| Colton | Co(a),Co(b) | Co(a),Co(b),Co(a-b-) | aqp1 | Co(b)<0.2% |
| Diego | Dia/Dib and Wra/Wrb | Dia,Dib,Wra,Wrb | SLC4A1 | Dia+ |
| Gerbich | Ge | Ge+,Ge- | Glycophorin C | Ge- |
| P | P1,P,Pk | P1,P2,P- | A4GALT | P2 |
| Scianna | Sc1,Sc2,Sc3,Sc4 | Sc1,Sc2,Sc3,Sc4 | ERMAP | Sc2,Sc4 |
| Yt(Cartwright) | Yta,Ytb | Yta,Ytb | ACHE | Ytb |
| Ii | I,i | I,i | IGNT | i |
| JR | JR | JR+,JR- | ABCG2 | JR- |
| Lan | Lan | Lan+,Lan- | ABCB6 | Lan- |

These and other changes can be made to the embodiments in light of the above-detailed description.

### Examples

### EXAMPLE 1

### Obtaining and gene targeting of hESCs

hESCs were obtained and expanded stably. Related assays and detections (Embryonic stem cell and multipotent markers, Karyotype, alkaline phosphotase etc.) were proceeded to ensure the appropriate states of human embryonic stem cells.

TALEN plasmids targeting gene H were designed as 2 left binding sequences paired with 3 right binding, as illustrated in Table 4.

**Table 4**

| | |
|---|---|
| L1: | cccctgctcttggactt |
| L2: | cccctgctcttggact |
| R1: | cccctgctcttggac |
| R2: | ctctgcggatctgtt |
| R3: | ctctgcggatctgt |

After converting binding sequencing into corresponding amino acid modules and DNA sequencing coding amino acid modules (see Tables 1 and 2). Then, TALEN plasmids were assembled and transfected into 293T cells to verify activities in pairs (L1R1, L1R2, L1R3, L2R1, L2R2, L2R3). Pair L1R1 caused considerably high efficiency of mutation, thus L1R1 was utilized to target gene H in hESCs.

### EXAMPLE 2

### Preparation of Gene H knockout hESCs stable line

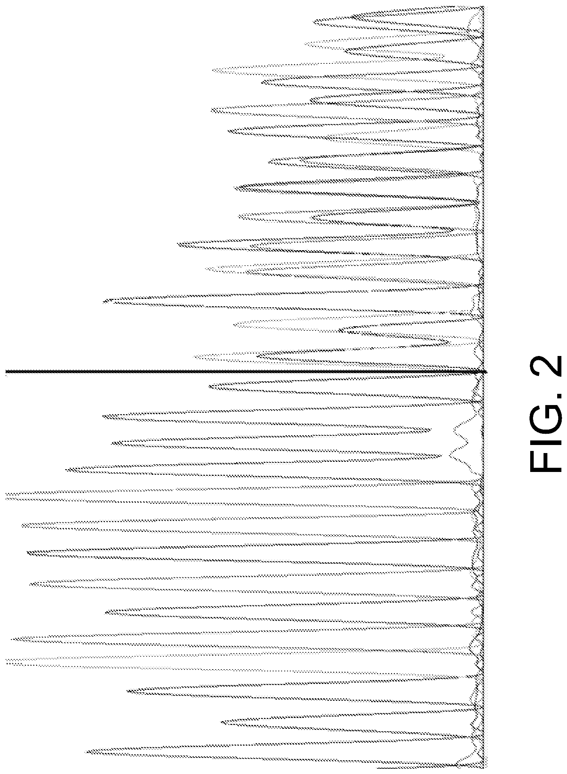

L1R1 plasmids were transfected into hESCs via related reagents. Puromycin was utilized to screen transfected cells for existence of puro-resistance gene in L1 plasmids. After screening, puromycin was withdrawn from media of hESCs to undergo recovery growth. Then the cell mix was collected to prepare genomics DNA. PCR-sequencing potential area of genomic DNA could verify whether the targeting of gene H succeeded. The mutation generated by TALEN could lead to 'peaks over peaks' in sequencing chromatogram, indicating the mixture of cells containing different genomic constitution (Figure 2). Then digested cell mix into single cells and plated into 96 well-plate. After the single cells grow to clone, collect enough sample to do PCR-sequencing again to confirm the successfully gene H knockout clones. The selected clone of hESCs were expanded and preserved.

### EXAMPLE 3

### Differentiation of Gene H knockout hESCs into erythrocytes and appraisal of erythrocytes

Genetically modified hESCs were culture without adhesion to form embryoid body (EB). Then EB was cultured in combination of cytokines to differentiate into hematopoietic progenitor cells and eventually into erythrocytes. FACS assay could detect nullified expression of H antigen on surface of erythrocytes, and sera of blood group O, A, B, AB could not agglutinate these erythrocytes, indicating that the erythrocytes originated from genetically modified hESCs could be adaptable for recipient of blood group O, A, B, AB, even Bombay type (H antigen absent).

## Claims

1. A method for generating modified stem cells containing a complete or partial gene deletion of gene H of the blood group antigen (BGA) biosynthesis or transportation pathway that renders the product of gene H non-functional or having reduced function with respect to the synthesis of the ABO blood group antigen, the method comprising:
providing collected stem cells containing a target polynucleotide associated with gene H of the blood group antigen (BGA) biosynthesis or transportation pathway;
introducing a transcription activator-like (TAL) effector nuclease into the stem cells to generate the modified stem cells;
isolating the modified stem cells; and
culturing in a culture media the modified stem cells containing a complete or partial gene deletion of gene H;
and wherein multiple TAL effector repeat sequences bind to the target polynucleotide of gene H of the BGA biosynthesis or transportation pathway.

2. A method for generating blood substitutes, comprising:
the method of claim 1 for generating modified stem cells;
and
differentiating the modified stem cells to generate a modified blood cell that has a reduced amount of the ABO blood group antigen as compared to a corresponding wild-type cell.

3. The method of claim 1 or 2, wherein the modified cell includes a genetically modified hematopoietic stem cell; a genetically modified erythroid progenitor cell; a genetically modified stem cell; a genetically modified embryonic stem cell; or a genetically modified induced pluripotent stem cell.

4. The method of claim 2, wherein the modified blood cell has
a reduced amount of an ABO blood group antigen as compared to a corresponding wild-type blood cell;
has reduced expression of gene H of the BGA biosynthesis or transportation pathway as compared to a corresponding wild-type blood cell;
accumulates a reduced amount of an ABO blood group antigen in the modified cell as compared to a corresponding wild-type cell;
has a reduced amount of an ABO blood group antigen on the modified cell as compared to a corresponding wild-type cell; or
has a reduced amount of an ABO blood group antigen associated with a membrane of the modified cell as compared to a corresponding wild-type cell.

5. The method of claim 2, wherein the modified blood cell has decreased immunogenicity as compared to a corresponding wild-type cell in response to a blood transfusion.

6. The method of claim 5, wherein the decreased immunogenicity comprises a decreased level of immunological responses induced by the modified cell as compared to the corresponding wild-type cell.

7. The method of claim 6, wherein the level of immunological responses induced by the modified cell is at least 20% of a level of immunological responses induced by the corresponding wild-type cell.

8. The method of claim 7, wherein the level of immunological responses induced by the modified cell is at least 50% of a level of immunological responses induced by the corresponding wild-type cell.

9. The method of claim 5, wherein the level of immunological responses induced by the modified stem cell or cells is less than a level of immunological responses induced by the corresponding wild-type cell.

10. The method of claim 5, wherein the level of immunological responses induced by the modified stem cell or cells is 1.5, 2, 3, 4, 5, 6, 7, 8, 9, 10, or 20 fold less.

11. The method of claim 5, wherein the level of immunological responses induced by the modified stem cell or cells is measured at day 0.5, 1, 2, 3, 4, 5, 6, 7, 8, 9, 10, 11, 12, 13, or 14 post-initiation of an injection of the modified cell; is measured at hour 0.5, 1, 2, 3, 4, 5, 6, 7, 8, 9, 10, 11, 12, 13, or 14 post-initiation of an injection of the modified cell; or is measured at minute 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 post-initiation of an injection of the modified cell.

## Patentansprüche

1. Ein Verfahren zur Erzeugung modifizierter Stammzellen, die eine vollständige oder partielle Gen-Deletion von Gen H der/des Blutgruppenantigen(BGA)-Biosynthese oder -Transportwegs enthält, welche das Produkt von Gen H funktionslos macht oder diesem eine verringerte Funktion verleiht, verglichen mit der Synthese des ABO-Blutgruppenantigens, wobei das Verfahren umfasst:
Bereitstellen gesammelter Stammzellen, die ein Ziel-Polynukleotid enthalten, das mit Gen H der/des Blutgruppenantigens (BGA)-Biosynthese oder -Transportweg assoziiert ist,
Einbringen einer Transkriptionsaktivator-artigen(TAL) Effektornuklease in die Stammzellen, um die modifizierten Stammzellen zu erzeugen,
Isolieren der modifizierten Stammzellen, und
Anzüchten der modifizierten Stammzellen, die eine vollständige oder partielle Gen-Deletion von Gen H enthalten, in einem Anzuchtmedium,
und wobei mehrere TAL-Effektor-Wiederholungssequenzen an das Ziel-Polynukleotid von Gen H der/des BGA-Biosynthese oder -Transportwegs binden.

2. Ein Verfahren zur Erzeugung von Blutersatzmitteln, umfassend:
das Verfahren nach Anspruch 1 zur Erzeugung modifizierter Stammzellen, und
das Differenzieren der modifizierten Stammzellen, um eine modifizierte Blutzelle zu erzeugen, die eine verringerte Menge des ABO-Blutgruppenantigens besitzt, verglichen mit einer entsprechenden Wildtypzelle.

3. Das Verfahren nach Anspruch 1 oder 2, wobei die modifizierte Zelle eine genetisch modifizierte hämatopoietische Stammzelle, eine genetisch modifizierte erythroide Vorläuferzelle, eine genetisch modifizierte Stammzelle, eine genetisch modifizierte embryonale Stammzelle oder eine genetisch modifizierte induzierte pluripotente Stammzelle einschließt.

4. Das Verfahren nach Anspruch 2, wobei die modifizierte Blutzelle
eine verringerte Menge eines ABO-Blutgruppenantigens besitzt, verglichen mit einer entsprechenden Wildtyp-Blutzelle,
eine verringerte Expression von Gen H der/des BGA-Biosynthese oder -Transportwegs besitzt, verglichen mit einer entsprechenden Wildtyp-Blutzelle,
eine verringerte Menge eines ABO-Blutgruppenantigens in der modifizierten Zelle ansammelt, verglichen mit einer entsprechenden Wildtypzelle,
eine verringerte Menge eines ABO-Blutgruppenantigens auf der modifizierten Zelle besitzt, verglichen mit einer entsprechenden Wildtypzelle, oder
eine verringerte Menge eines ABO-Blutgruppenantigens, assoziiert mit einer Membran der modifizierten Zelle, besitzt, verglichen mit einer entsprechenden Wildtypzelle.

5. Das Verfahren nach Anspruch 2, wobei die modifizierte Blutzelle eine verringerte Immunogenizität besitzt, verglichen mit einer entsprechenden Wildtypzelle, in Reaktion auf eine Bluttransfusion.

6. Das Verfahren nach Anspruch 5, wobei die verringerte Immunogenizität ein verringertes Level an immunologischen Reaktionen, die durch die modifizierte Zelle hervorgerufen werden, umfasst, verglichen mit der entsprechenden Wildtypzelle.

7. Das Verfahren nach Anspruch 6, wobei das Level an immunologischen Reaktionen, die durch die modifizierte Zelle hervorgerufen werden, wenigstens 20% eines Levels an immunologischen Reaktionen, die durch die entsprechende Wildtypzelle hervorgerufen werden, beträgt.

8. Das Verfahren nach Anspruch 7, wobei das Level an immunologischen Reaktionen, die durch die modifizierte Zelle hervorgerufen werden, wenigstens 50% eines Levels an immunologischen Reaktionen, die durch die entsprechende Wildtypzelle hervorgerufen werden, beträgt.

9. Das Verfahren nach Anspruch 5, wobei das Level an immunologischen Reaktionen, die durch die modifizierte(n) Stammzelle(n) hervorgerufen werden, geringer ist als ein Level an immunologischen Reaktionen, die durch die entsprechende Wildtypzelle hervorgerufen werden.

10. Das Verfahren nach Anspruch 5, wobei das Level an immunologischen Reaktionen, die durch die modifizierte(n) Stammzelle(n) hervorgerufen werden, 1,5, 2, 3, 4, 5, 6, 7, 8, 9, 10 oder 20 Mal geringer ist.

11. Das Verfahren nach Anspruch 5, wobei das Level an immunologischen Reaktionen, die durch die modifizierte(n) Stammzelle(n) hervorgerufen werden, an Tag 0,5, 1, 2, 3, 4, 5, 6, 7, 8, 9, 10, 11, 12, 13 oder 14 nach der Initiierung einer Injektion der modifizierten Zelle gemessen wird; 0,5, 1, 2, 3, 4, 5, 6, 7, 8, 9, 10, 11, 12, 13 oder 14 Stunden nach der Initiierung einer Injektion der modifizierten Zelle gemessen wird; oder 1, 2, 3, 4, 5, 6, 7, 8, 9, 10, 11, 12, 13, 14, 15, 16, 17, 18, 19, 20, 21, 22, 23, 24, 25, 26, 27, 28, 29, oder 30 Minuten nach der Initiierung einer Injektion der modifizierten Zelle gemessen wird.

## Revendications

1. Procédé pour générer des cellules souches modifiées contenant une délétion génique complète ou partielle du gène H de la voie de biosynthèse ou de transport d'un antigène de groupe sanguin (BGA) qui rend le produit du gène H non fonctionnel ou lui confère une fonction réduite par rapport à la synthèse de l'antigène de groupe sanguin ABO, le procédé comprenant :
la mise à disposition de cellules souches collectées contenant un polynucléotide cible associé au gène H de la voie de biosynthèse ou de transport d'un antigène de groupe sanguin (BGA) ;
l'introduction d'une nucléase effectrice de type activateur de la transcription (TAL) dans les cellules souches afin de générer les cellules souches modifiées ;
l'isolement des cellules souches modifiées ; et
la mise en culture, dans un milieu de culture, des cellules souches modifiées contenant une délétion génique complète ou partielle du gène H ;
et dans lequel de multiples séquences de répétition effectrices TAL se lient au polynucléotide cible du gène H de la voie de biosynthèse ou de transport d'un BGA.

2. Procédé pour générer des substituts sanguins, comprenant :
le procédé selon la revendication 1 pour générer des cellules souches modifiées ;
et
la différenciation des cellules souches modifiées afin de générer une cellule sanguine modifiée qui présente une quantité réduite d'antigène de groupe sanguin ABO par comparaison à une cellule de type sauvage correspondante.

3. Procédé selon la revendication 1 ou 2, dans lequel la cellule modifiée comprend une cellule souche hématopoïétique génétiquement modifiée ; une cellule progénitrice érythroïde génétiquement modifiée ; une cellule souche génétiquement modifiée ; une cellule souche embryonnaire génétiquement modifiée ; ou une cellule souche pluripotente induite génétiquement modifiée.

4. Procédé selon la revendication 2, dans lequel la cellule sanguine modifiée
présente une quantité réduite d'un antigène de groupe sanguin ABO par comparaison à une cellule sanguine de type sauvage correspondante ;
présente une expression réduite du gène H de la voie de biosynthèse ou de transport d'un BGA par comparaison à une cellule sanguine de type sauvage correspondante ;
accumule une quantité réduite d'un antigène de groupe sanguin ABO dans la cellule modifiée par comparaison à une cellule de type sauvage correspondante ;
présente une quantité réduite d'un antigène de groupe sanguin ABO sur la cellule modifiée par comparaison à une cellule de type sauvage correspondante ; ou
présente une quantité réduite d'un antigène de groupe sanguin ABO associé à une membrane de la cellule modifiée par comparaison à une cellule de type sauvage correspondante.

5. Procédé selon la revendication 2, dans lequel la cellule sanguine modifiée présente une immunogénicité réduite par comparaison à une cellule de type sauvage correspondante en réponse à une transfusion sanguine.

6. Procédé selon la revendication 5, dans lequel l'immunogénicité réduite comprend un taux réduit de réponses immunologiques induites par la cellule modifiée par comparaison à la cellule de type sauvage correspondante.

7. Procédé selon la revendication 6, dans lequel le taux de réponses immunologiques induites par la cellule modifiée est au moins 20 % d'un taux de réponses immunologiques induites par la cellule de type sauvage correspondante.

8. Procédé selon la revendication 7, dans lequel le taux de réponses immunologiques induites par la cellule modifiée est au moins 50 % d'un taux de réponses immunologiques induites par la cellule de type sauvage correspondante.

9. Procédé selon la revendication 5, dans lequel le taux de réponses immunologiques induites par la ou les cellules souche(s) modifiée(s) est inférieur à un taux de réponses immunologiques induites par la cellule de type sauvage correspondante.

10. Procédé selon la revendication 5, dans lequel le taux de réponses immunologiques induites par la ou les cellules souche(s) modifiée(s) est 1,5, 2, 3, 4, 5, 6, 7, 8, 9, 10, ou 20 moins moindre.

11. Procédé selon la revendication 5, dans lequel le taux de réponses immunologiques induites par la ou les cellules souche(s) modifiée(s) est mesuré à 0,5, 1, 2, 3, 4, 5, 6, 7, 8, 9, 10, 11, 12, 13, ou 14 jour(s) post-initiation d'une injection de la cellule modifiée ; est mesuré à 0,5, 1, 2, 3, 4, 5, 6, 7, 8, 9, 10, 11, 12, 13, ou 14 heure(s) post-initiation d'une injection de la cellule modifiée ; ou est mesuré à 1, 2, 3, 4, 5, 6, 7, 8, 9, 10, 11, 12, 13, 14, 15, 16, 17, 18, 19, 20, 21, 22, 23, 24, 25, 26, 27, 28, 29, ou 30 minute(s) post-initiation d'une injection de la cellule modifiée.
